# EUROPÄISCHE PATENTSCHRIFT

(11) **EP 2 946 826 B1**
(45) Veröffentlichungstag und Bekanntmachung des Hinweises auf die Patenterteilung: **27.03.2019**
(21) Anmeldenummer: 15166625.2
(22) Anmeldetag: 06.05.2015
(51) Int. Cl.: B01D 53/04, A61M 16/00, B01D 53/02

(54) **FILTERANLAGE FÜR EIN GEBÄUDE UND FILTEREINRICHTUNG**
FILTER SYSTEM FOR A BUILDING AND FILTER DEVICE
INSTALLATION DE FILTRE POUR UN BATIMENT ET DISPOSITIF DE FILTRE

(30) Priorität: 21.05.2014 DE 202014102383 U
(43) Veröffentlichungstag der Anmeldung: 25.11.2015
(73) Patentinhaber: ZeoSys GmbH, 13088 Berlin (DE)
(72) Erfinder: Welke, Hartmut, 16356 Ahrensfelde (DE)
(74) Vertreter: Schulz Junghans Patentanwälte PartGmbB

(56) Entgegenhaltungen:
- US-A1- 2009 249 954
- US-A1- 2012 222 556
- US-A1- 2013 220 330

## Beschreibung

Die Erfindung betrifft eine Filteranlage für ein Gebäude, insbesondere ein Krankenhaus, und eine Filtereinrichtung.

### Hintergrund

Filter für halogenierte Kohlenwasserstoffe (HKW) sind aus dem Stand der Technik bekannt. Reaktiv wirkende Aktivkohlen sind für die Reinigung von Prozess- oder Abluft geeignet. Derartige Aktivkohlen sind in den Dokumenten DE 37 13 346 A1, DE 39 35 094 A1 und DE 40 03 668 A1 beschrieben. Die Voraussetzungen für hohe Sorptionskapazität verbunden mit optimaler Regenerierbarkeit sind in den Schriften DD 239 947, DE 36 28 858 A1 und DE 37 31 688 A1 dargelegt.

Des Weiteren ist bekannt, als Sorbentien den Verfahren angepasste dealuminierte Zeolithe zu verwenden, wie in Dokument DE 197 49 963 A1 beschrieben ist. Die sorbierten HKW werden durch Erwärmen desorbiert, kondensiert und wiedergewonnen. In Dokument DE 101 18 768 A1 ist für eine Filterpatrone die schonende Regenerierung mit einem Wasserdampfträger beschrieben. Modifizierte und / oder dealuminierte Zeolithe mit geringer Wasseraufnahme unterhalb 2 Masseprozent bewirken ein das Sorbens und das Sorbat schonendes Absenken der Desorptionstemperatur.

In dem Dokument DE 10 2006 008 320 A1 ist ein Gasdurchtritt im oberen Wandbereich eines einzelnen Filtereinsatzes so ausgestaltet, dass in diesem Bereich eine Pfropfenströmung für die Gase entsteht, wobei eine Vergleichmäßigung der Durchbruchkurven für Inhalationsanästhetika erzielt wird. Die eingesetzten Anästhesiegase weisen somit am oberen Rand des Filtereinsatzes mit den hydrophoben Zeolithen "gute" Durchbruchskurven auf, das heißt mit einer steilen und klar örtlich sowie zeitlich bestimmten Charakteristik des Übergangs, indem sich eine scharfe Grenze zwischen den beladenen und noch unbeladenen Teilen der Zeolithschüttung ausbildet. Das in der Wirkung komplexe Filtersystem ist unübersichtlich und lässt für seinen zeitlichen Ablauf eine mögliche Ermittlung von Bestwerten nur aufgrund eines langfristigen und empirischen Gewinns an Arbeitserfahrung zu.

Es hat nicht an Versuchen gefehlt, den Beladungsgrad von Sorptionsbetten bei der Reinigung von Gasströmen zu erhöhen. In dem Dokument DE 43 19 327 A1 wird ein Rohgasstrom aufeinanderfolgend durch zwei Sorptionsbetten geleitet. Nach Beendigung des Prozesses wird das erste Sorbensbett regeneriert und die Strömungsrichtung umgekehrt, so dass das zweite Bett zuerst durchströmt wird. Dabei wird jedoch umständlich das gebrauchte Sorbens durch ein frisch regeneriertes ausgetauscht.

Es ist auch bekannt, dass eine Verbesserung in der sorptiven Trennung durch physikalische Unterschiede ermöglicht wird, beispielsweise in den Porengrößen und durch Veränderungen an den Sorbensbetten selbst. Bei ungleichen Gaskomponenten, wie sie in Dokument DE 197 06 806 A1 die Gemischanteile von Lachgas und Narkosemitteldämpfen bilden, kann deren selektive Trennung durch verschiedenartige Molekularsiebtypen erfolgen.

In der Druckschrift WO 2009/083275 A1 wird ein erstes Sorptionsbett mit einer hydrophoben Molekularsiebkohle vor ein zweites Sorptionsbett mit einem hydrophoben Zeolithen in Reihe (hintereinander) verschaltet. Beide stellen jeweils eine Prozess-Stufe dar und werden vom Sorptiv räumlich aufeinanderfolgend durchströmt. Beide Stufen weisen einen gemeinsamen Durchsatzparameter für das Trägergas, vor allem für Luft wie auch bei der Regeneration für das Regenerationsmittel Wasserdampf auf. Diese Prozessgrößen sind naturgemäß vom Gasdruck und von der Temperatur sowie vom Gasmengenstrom des Trägergases abhängig. Die Filteranordnung kann in einer Produktions- oder Gasreinigungsanlage kontinuierlich wirken oder auch als regenerierbare und zweckmäßig evakuierbare Filterpatrone ausgestaltet sein. Es wird eine Sorption aus dem Trägergas, insbesondere eine Desorption in das Trägergas und eine Destillation mit Sattdampf miteinander kombiniert.

Das Dokument US 2013/0220330 A1 offenbart eine Vorrichtung zum Sammeln und Wiederverwerten von Narkosegasen. Das Narkosegas kann aus mehreren Quellen eingesammelt und einer zentralen Verwertungsanlage zugeführt werden.

Das Dokument US 2012/0222556 A1 offenbart die Merkmale des Oberbegriffs von Anspruch 1.

Das Dokument US 2009/0249954 A1 offenbart ein Verfahren zum Durchströmen eines monolithischen Sorbats zur Sorption von Spuren von Fremdstoffen aus einem Fluidstrom.

Trotz der Vielzahl von bekannten Filtern und Filtereinrichtungen gehen in Krankenhäusern üblicherweise Narkosegase über eine zentrale Absauganlage (Rohrleitung) ungefiltert in die freie Atmosphäre und führen zu einer Belastung der Umwelt.

### Zusammenfassung

Aufgabe ist es, verbesserte Technologien zum Reinigen der Abluft von Gebäuden anzugeben.

Die Aufgabe wird durch die Filteranlage nach Anspruch 1 gelöst. Weitere Ausführungsformen sind Gegenstand von abhängigen Ansprüchen.

Nach einem Aspekt ist eine Filteranlage für ein Gebäude, insbesondere ein Krankenhaus, bereitgestellt. Die Filteranlage weist einen Abluftschacht auf, mittels dem ein Gasgemisch aus dem Gebäude herausgeführt wird. Des Weiteren weist die Filteranlage eine dem Abluftschacht zugeordnete Filtereinrichtung auf, die ausgelegt ist, Narkosegase aus dem die Filtereinrichtung durchströmenden Gasgemisch wenigstens teilweise heraus zu filtern. Die Filtereinrichtung kann in dem Abluftschacht angeordnet sein. Alternativ kann die Filtereinrichtung vor oder hinter dem Abluftschacht angeordnet sein. Die Filteranlage kann mehrere Filtereinrichtungen aufweisen.

Das Gasgemisch kann beispielsweise Luft sein (also Stickstoff, Sauerstoff, Kohlenstoffdioxid und Wasserdampf enthalten) die mit einem oder mehreren Narkosegasen (Inhalationsanästhetika) versetzt ist. Beispiele für Narkosegase sind Lachgas, Sevofluran, Enfluran, Isofluran, Haloethan und Desfluran. Die Filtereinrichtung ist ausgelegt, das oder die Narkosegase wenigstens teilweise aus dem Gasgemisch zu entfernen.

Die Filteranlage ist insbesondere zur Installation in einem Krankenhaus geeignet. Derzeit werden etwa 6 Millionen Liter von Narkosegasen in die freie Atmosphäre abgegeben. Mittels der Filteranlage können Narkosegase aus der Luft herausgefiltert werden, sodass sie nicht in die Umwelt gelangen. Dies vermindert zum einen den Treibhauseffekt. Darüber hinaus können die gefilterten Narkosegase aus der Filtereinrichtung wiedergewonnen und nach einer Aufbereitung erneut eingesetzt werden.

Es kann vorgesehen sein, dass in dem Gebäude Luft aus mehreren Räumen, beispielsweise Operationssälen, zusammengeführt und durch den Abluftschacht aus dem Gebäude herausgeführt wird. Die mehreren Räume können mit einem Anästhesiegas-Fortleitungssystem verbunden sein. Es ist die Möglichkeit geschaffen, mit einer zentralen Filteranlage die Abluft aus mehreren Räumen zu filtern.

Der Durchfluss des Gasgemisches durch die Filtereinrichtung kann zwischen 40 bis 60 l/min betragen.

Die Filtereinrichtung weist mehrere Filter auf. Die Filter können zumindest teilweise mit Aktivkohle und / oder einem Zeolith gefüllt sein. Die Aktivkohle und / oder das Zeolith bilden ein Sorptionsmittel, an welchem das Narkosegas adsorbiert. Das Sorptionsmittel kann mikroporös sein. Zeolithe sind kristalline Alumosilikate, die in zahlreichen Modifikationen in der Natur vorkommen, aber auch synthetisch hergestellt werden können. Beispielsweise können Si-reiche (dealuminierte) Zeolithe als Sorptionsmittel (Filtermaterial) verwendet werden, bevorzugt mit einem Si:Al Verhältnis von größer als 180:1 (entspricht einem SiO₂:Al₂O₃ Verhältnis von 360:1). Mehrere Filter können hintereinander und / oder nebeneinander angeordnet sein. Es kann eine einzelne Reihe von mehreren Filtern gebildet sein. Alternativ kann eine zweidimensionale (matrixartige) Anordnung von Filtern vorgesehen sein, wo beispielsweise zwei Reihen mit je vier Filtern gebildet sind.

Eine erste Messeinrichtung ist der Filtereinrichtung vorgeschaltet, wobei die erste Messeinrichtung konfiguriert ist, eine Menge von Narkosegas in dem in die Filtereinrichtung einströmenden Gasgemisch zu bestimmen. Des Weiteren kann eine zweite Messeinrichtung der Filtereinrichtung nachgeschaltet sein, wobei die zweite Messeinrichtung konfiguriert ist, eine Menge von Narkosegas in dem aus der Filtereinrichtung ausströmenden Gasgemisch zu bestimmen. Alternativ oder ergänzend können die erste und / oder zweite Messeinrichtung konfiguriert sein, eine Konzentration von Narkosegas in dem Gasgemisch zu bestimmen. Die erste und / oder zweite Messeinrichtung können eine Anzeigeeinrichtung aufweisen, um Messwerte anzuzeigen.

Die Filteranlage weist eine Steuereinrichtung auf, die konfiguriert ist, abhängig von der mittels der ersten Messeinrichtung ermittelten Narkosegasmenge eine Anzahl von Filtern einzustellen, welche das Gasgemisch beim Durchströmen der Filtereinrichtung passiert. Die Steuereinrichtung kann einen Prozessor, einen Arbeitsspeicher, ein Speichermedium und / oder Ein- sowie Ausgänge für Signale aufweisen.

Jeder Filter der Filtereinrichtung kann eine Füllstandsmesseinrichtung aufweisen, die konfiguriert ist, einen Füllstand des jeweiligen Filters mit Narkosegas zu bestimmen und ggf. anzuzeigen.

Gemäß einer Weiterbildung kann die Filtereinrichtung eine Filteraufnahme zum Aufnehmen eines oder mehrerer von dem Gasgemisch zu durchströmenden Filtern, ein Magazin zum Vorhalten von unverbrauchten Filtern sowie ein Lager zum Aufnehmen von verbrauchten Filtern aufweisen. Verbrauchte Filter können aus dem Lager entfernt und einer Wiedergewinnungseinrichtung zugeführt werden. Neue, unverbrauchte Filter können in das Magazin eingebracht werden. Die Entnahme / Eingabe der Filter kann im laufenden Betrieb der Filteranlage erfolgen.

Die Filteranlage kann in einer weiteren Ausführungsform eine Wechseleinrichtung aufweisen, wobei die Steuereinrichtung weiter konfiguriert ist, einen verbrauchten Filter mittels der Wechseleinrichtung von der Filteraufnahme in das Lager und einen unverbrauchten Filter von dem Magazin in die Filteraufnahme zu transportieren. Ein Austausch der Filter kann während des Betriebs der Filteranlage durchgeführt werden.

Die Filtereinrichtung kann beispielsweise einen Vorfilter zum Filtern von Partikeln und / oder Feuchtigkeit aus dem Gasgemisch aufweisen. Hiermit kann Staub und / oder Wasserdampf aus dem Gasgemisch entfernt werden, bevor das Gasgemisch in die Filtereinrichtung eintritt. Die Filtereinrichtung kann des Weiteren einen Auffangbehälter zum Aufnehmen des gefilterten Wasserdampfs aufweisen.

### Beschreibung beispielhafter Ausführungsformen

Im Folgenden werden beispielhafte Ausführungsformen unter Bezugnahme auf Figuren einer Zeichnung näher erläutert. Hierbei zeigen:
- Fig. 1: eine schematische Darstellung eines Gebäudes mit einer Filteranlage,
- Fig. 2: eine Ausführungsform der Filteranlage mit einer Filtereinrichtung,
- Fig. 3: eine weitere Ausführungsform der Filtereinrichtung und
- Fig. 4: zeigt beispielhaft für einen Filter eine andere Weiterbildung der Filtereinrichtung.

Im Folgenden werden für gleiche Komponenten gleiche Bezugszeichen verwendet.

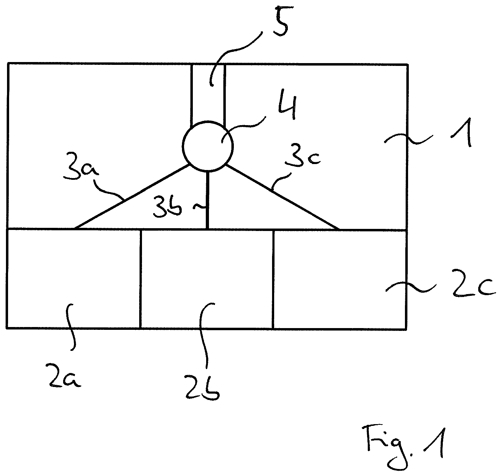

In Fig. 1 ist schematisch ein Gebäude 1 mit mehreren Räumen 2a, 2b, 2c dargestellt. Aus jedem Raum 2a, 2b, 2c wird die Luft abgesaugt und gelangt mittels Leitungen 3a, 3b, 3c in eine Filtereinrichtung 4. Die Filtereinrichtung 4 ist einem Schacht 5 vorgelagert und ausgelegt, Narkosegase wenigstens teilweise aus der Luft zu filtern. Nachdem die Luft die Filtereinrichtung 4 durchströmt hat, ist diese von Narkosegasen gereinigt. Die gereinigte Luft verlässt das Gebäude 1. Mit der Filtereinrichtung 4 ist ein zentraler Filter für die Abluft der Räume 2a, 2b, 2c bereitgestellt.

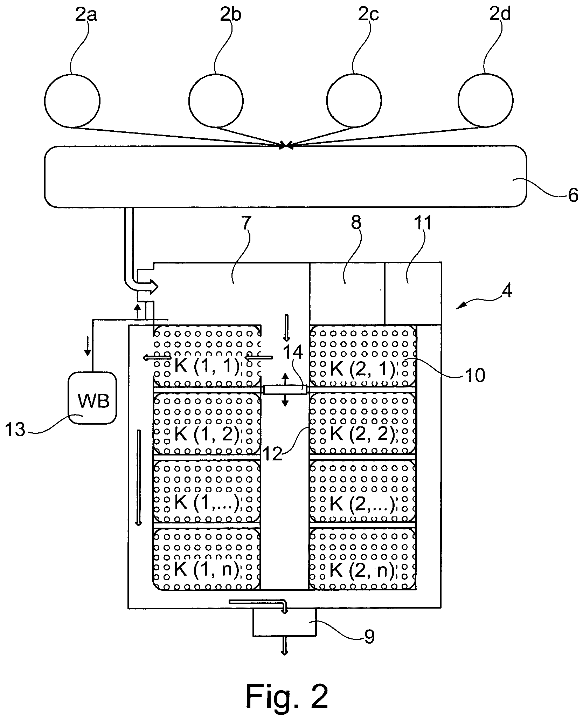

Fig. 2 zeigt eine Ausführungsform einer Filteranlage. Luft aus den Operationsräumen 2a, 2b, 2c, 2d wird mittels eines Anästhesiegas-Fortleitungssystem 6 gesammelt. Die Luft ist ein Gasgemisch, das Stickstoff, Sauerstoff, Kohlenstoffdioxid, Wasserdampf enthält und mit Narkosegasanteilen versetzt ist. Des Weiteren kann die Luft Feinpartikel aufweisen, beispielsweise Staub. Die Luft wird in die Filtereinrichtung 4 eingeleitet. Die Luft wird zunächst durch einen Vorfilter 7 geführt. Mit dem Vorfilter 7 werden Partikel und / oder Wasserdampf aus der Luft gefiltert. Das gefilterte Wasser wird in einem Behälter 13 aufgefangen.

Nachdem die Luft den Vorfilter 7 passiert hat, wird sie mittels eines ersten Gassensors 8 untersucht. Der Gassensor 8 ist konfiguriert, die Menge und / oder Konzentration des Narkosegases (oder der Narkosegase) in der Luft zu bestimmen. Die Messwerte werden an eine Steuereinrichtung 11 übermittelt. Abhängig von den ermittelten Messwerten ermittelt die Steuereinrichtung 11, durch wie viele Filter 10 die Luft strömen muss, um einen vorgegebenen Wert an Narkosegasmenge und / oder Narkosegaskonzentration in der ausströmenden Luft zu erhalten. Bei der Ermittlung kann der Füllstand der Filter berücksichtigt werden. Die Steuereinrichtung ermittelt geeignete Einstellungen für Verschlusselemente 12, 14, mit welchen ein Weg der Luft durch die Filtereinrichtung eingestellt werden kann. Das Verschlusselement 14 ist vertikal verstellbar. Darüber hinaus können die Verschlusselemente 12 der Filter 10 geöffnet oder geschlossen werden, um einen Eintritt der Luft in den jeweiligen Filter zu ermöglichen bzw. zu verhindern. Bei der gezeigten Ausführungsform sind die Verschlusselemente 12, 14 derart eingestellt, dass die Luft nur durch einen Filter (K(1,1)) geführt wird. Andere Kombinationen der Verschlusselemente 12, 14 sind möglich, um weitere Filter 10 in den Reinigungsprozess einzubeziehen.

An einem Luftaustritt der Filereinrichtung 4 ist ein zweiter Gassensor 9 angeordnet. Dieser ermittelt die Narkosegasmenge und / oder Narkosegaskonzentration in der aus der Filtereinrichtung ausströmenden Luft. Die Messwerte können zur Kontrolle an die Steuereinrichtung 11 übermittelt werden. Abhängig von den Messwerten kann ggf. die Anzahl an durchströmten Filtern 10 angepasst werden, um einen vorgegebenen Messwert zu erzielen.

Die Filter 10 sind mit dealuminierten Zeolithen oder Aktivkohle gefüllt, an welchen das Narkosegas sorbiert.

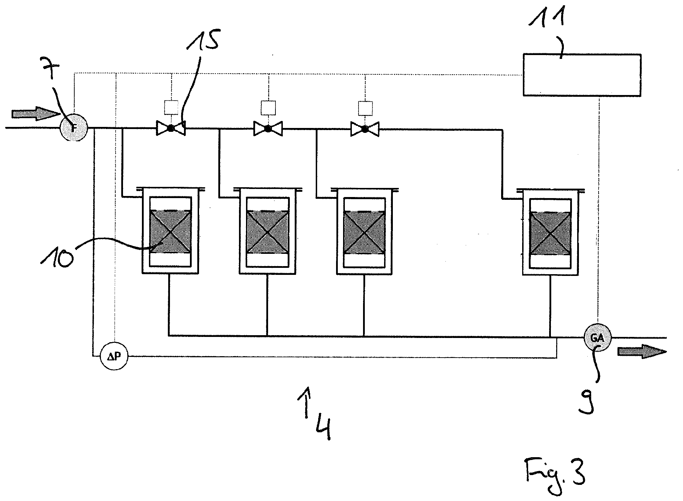

Fig. 3 zeigt eine andere Ausführungsform der Filtereinrichtung 4. Am Eintritt ist ein Vorfilter 7 angeordnet, der zunächst Staub und Wasser entfernt. Der Vorfilter 7 kann mit einem Gassensor gekoppelt sein, um die Narkosegasmenge und / oder Narkosegaskonzentration zu bestimmen. Die Werte können dann an die Steuereinrichtung 11 übermittelt werden.

Mehrere Filter 10 sind parallel zueinander geschaltet. Die Steuereinrichtung 11 bestimmt (abhängig von den übermittelten Werten), welche Ventile 15 geöffnet oder geschlossen werden, um die Anzahl der von Luft durchströmten Filter einzustellen.

Am Austritt der Filtereinrichtung 4 ist der zweite Gassensor 9 zur Kontrolle des ausströmenden Gasgemischs vorgesehen.

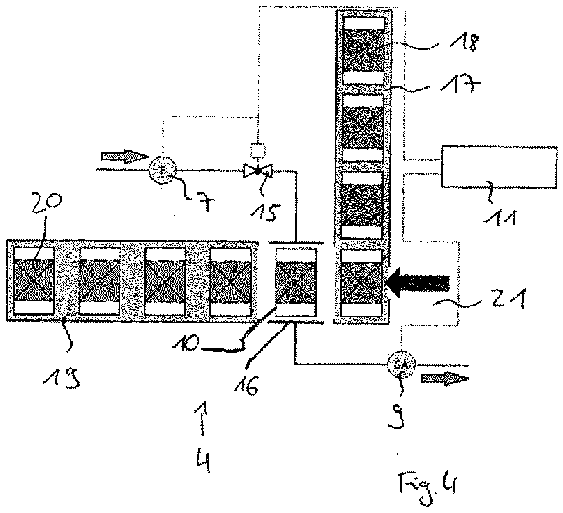

In Fig. 4 ist eine weitere Ausführungsform dargestellt. Einströmende Luft wird zunächst von dem Vorfilter 7 von Staub und Wasser gereinigt. Auch in diesem Fall ist der Vorfilter 7 mit einem Gassensor zur Analyse des Gasgemisches gebildet. Messwerte zur Menge und / oder Konzentration des Narkosegases werden an die Steuereinrichtung 11 übertragen. Die Steuereinrichtung 11 regelt das Ventil 15, um die Strömungsmenge der Luft einzustellen.

Die Luft strömt durch den Filter 10. Der Filter 10 ist in einer Filteraufnahme 16 angeordnet, welche den Filter 10 im Gasströmungsweg hält. Der Filter 10 kann in dieser Ausführungsform auch als "aktiver Filter" bezeichnet werden. Die Filtereinrichtung umfasst ein Magazin 17 und ein Lager 19. In dem Magazin 17 sind neue, unverbrauchte Filter angeordnet, die zum Einsatz kommen, wenn der aktive Filter 10 voll ist. Alle Filter sind mit einem Füllstandssensor ausgestattet (nicht dargestellt). Die Daten des Füllstandssensor des aktiven Filters 10 werden an die Steuereinrichtung übermittelt. Wenn der aktive Filter 10 voll ist, wird mittels einer Wechseleinrichtung 21 ein neuer Filter 18 aus dem Magazin 17 in die Filteraufnahme gebracht. Der verbrauchte Filter 10 wird in das Lager 19 transportiert, wo er mit anderen verbrauchten Filtern 20 vorübergehend gelagert wird. Die verbrauchten Filter 20 können aus dem Lager 19 entnommen werden. Sie können einem Regenerierungsprozess zugeführt werden, um das gefilterte Narkosegas zurückzugewinnen. Ein Verfahren zur Rückgewinnung von Narkosegasen ist in dem Dokument WO 2009/083275 A1 beschrieben. Neue Filter 18 können dem Magazin zugeführt werden. Der Wechsel des aktiven Filters 10 erfolgt im laufenden Betrieb der Anlage.

Am Ausgang der Filtereinrichtung 4 ist der Gassensor 9 gebildet, mit welchem das ausströmende Gasgemisch auf Narkosegas kontrolliert wird.

Es ist möglich, die in Fig. 3 und 4 gezeigten Ausführungsformen miteinander zu kombinieren, derart, dass jeder der parallel geschalteten Filter mit einem Magazin und einem Lager sowie einer Wechseleinrichtung gekoppelt ist.

Die in der Beschreibung, den Ansprüchen und den Figuren offenbarten Merkmale können sowohl einzeln als auch in beliebiger Kombination miteinander für die Verwirklichung der in Anspruch 1 definierten Erfindung relevant sein.

## Patentansprüche

1. Filteranlage für ein Gebäude (1), insbesondere ein Krankenhaus, mit
- einem Abluftschacht (5), mittels dem ein Gasgemisch aus dem Gebäude (1) heraus geführt wird,
- einer dem Abluftschacht (5) zugeordneten Filtereinrichtung (4), die ausgelegt ist, Narkosegase aus dem die Filtereinrichtung (4) durchströmenden Gasgemisch wenigstens teilweise heraus zu filtern, wobei die Filtereinrichtung (4) mehrere Filter (10) aufweist, und
- einer ersten Messeinrichtung (8), die der Filtereinrichtung (4) vorgeschaltet ist, und konfiguriert ist, eine Menge von Narkosegas in dem in die Filtereinrichtung (4) einströmenden Gasgemisch zu bestimmen,
**dadurch gekennzeichnet, dass**
- die Filteranlage weiterhin eine Steuereinrichtung (11) aufweist, die konfiguriert ist, abhängig von der mittels der ersten Messeinrichtung (8) ermittelten Narkosegasmenge eine Anzahl von Filtern (10) einzustellen, welche das Gasgemisch beim Durchströmen der Filtereinrichtung (4) passiert.

2. Filteranlage nach Anspruch 1, wobei jeder Filter (10) der Filtereinrichtung eine Füllstandsmesseinrichtung aufweist, die konfiguriert ist, einen Füllstand des jeweiligen Filters mit Narkosegas zu bestimmen.

3. Filteranlage nach einem der vorangehenden Ansprüche, wobei die Filtereinrichtung (4) eine Filteraufnahme (16) zum Aufnehmen eines oder mehrerer von dem Gasgemisch zu durchströmenden Filtern (10), ein Magazin (17) zum Vorhalten von unverbrauchten Filtern (18) sowie ein Lager (19) zum Aufnehmen von verbrauchten Filtern (20) aufweist.

4. Filteranlage nach den Ansprüchen 1 bis 3, weiter eine Wechseleinrichtung (21) aufweisend, wobei die Steuereinrichtung (11) weiter konfiguriert ist, einen verbrauchten Filter (20) mittels der Wechseleinrichtung (21) von der Filteraufnahme (16) in das Lager (19) und einen unverbrauchten Filter (18) von dem Magazin (17) in die Filteraufnahme (16) zu transportieren.

5. Filteranlage nach einem der vorangehenden Ansprüche, wobei eine zweite Messeinrichtung (9) der Filtereinrichtung (4) nachgeschaltet ist und die zweite Messeinrichtung (9) konfiguriert ist, eine Menge von Narkosegas in dem aus der Filtereinrichtung (4) ausströmenden Gasgemisch zu bestimmen.

6. Filteranlage nach einem der vorangehenden Ansprüche, wobei die Filtereinrichtung (4) einen Vorfilter (7) zum Filtern von Partikeln und / oder Feuchtigkeit aus dem Gasgemisch aufweist.

## Claims

1. Filter system for a building (1), in particular a hospital, with
- an exhaust air shaft (5) by means of which a gas mixture is led out of the building (1),
- a filter device (4) associated with the exhaust air shaft (5), which is configured to at least partially filter anaesthetic gases out of the gas mixture streaming through the filter device (4), wherein the filter device (4) comprises a plurality of filters (10), and
- a first measuring device (8), which is arranged upstream of the filter device (4), and is configured to determine an amount of anesthetic gas in the gas mixture streaming into the filter device (4),
**characterized in that**
- the filter system further comprises a control device (11), which is configured to set a number of filters (10), which the gas mixture is passing by streaming through the filter device (4) depending on the quantity of anesthetic gas determined by the first measuring device (8).

2. Filter system according to claim 1, wherein each filter (10) of the filter system comprises a filling level measuring device configured to determine an anesthetic gas filling level of the respective filter.

3. Filter system according to any one of the preceding claims, wherein the filter device (4) comprises a filter receptacle (16) for receiving one or more filters (10) to be streamed through by the gas mixture, a magazine (17) for retaining unused filters (18) and a storage (19) for receiving of used filters (20).

4. Filter system according to claims 1 to 3, further comprising a changing device (21), wherein the control device (11) is further configured to transport a used filter (20) by means of the changing device (21) from the filter receptacle (16) into the storage (19) and an unused filter (18) from the magazine (17) into the filter receptacle (16).

5. Filter system according to any one of the previous claims, wherein a second measuring device (9) is connected downstream of the filter device (4) and the second measuring device (9) is configured to determine a quantity of anesthetic gas in the gas mixture streaming out of the filter device (4).

6. Filter system according to any one of the preceding claims, wherein the filter device (4) comprises a prefilter (7) for filtering particles and/or moisture from the gas mixture.

## Revendications

1. Installation de filtre pour un bâtiment (1), en particulier un hôpital, avec
- un puits d'air d'échappement (5) au moyen duquel un mélange de gaz est acheminé hors du bâtiment (1),
- un dispositif de filtre (4) associé au puits d'air d'échappement (5), qui est configuré pour filtrer au moins partiellement des gaz anesthésiques hors du mélange de gaz circulant à travers le dispositif de filtre (4), le dispositif de filtre (4) comprenant une pluralité de filtres (10), et
- un premier dispositif de mesure (8), qui est agencé en amont du dispositif de filtre (4), et
est configuré pour déterminer une quantité de gaz anesthésique dans le mélange de gaz circulant dans le dispositif de filtre (4),
**caractérisée par le fait que**
- l'installation de filtre comprend en outre un dispositif de commande (11), qui est configuré pour définir un nombre de filtres (10) à travers lesquels le mélange de gaz passe en circulant à travers le dispositif de filtre (4) en fonction de la quantité de gaz anesthésique déterminée par le premier dispositif de mesure (8).

2. Installation de filtre selon la revendication 1, dans laquelle chaque filtre (10) de l'installation de filtre comprend un dispositif de mesure de niveau de remplissage configuré pour déterminer un niveau de remplissage de gaz anesthésique du filtre respectif.

3. Installation de filtre selon l'une quelconque des revendications précédentes, dans laquelle le dispositif de filtre (4) comprend un réceptacle de filtre (16) pour recevoir un ou plusieurs filtres (10) à travers lesquels le mélange de gaz est amené à circuler, un magasin (17) pour retenir des filtres non usagés (18), et un dispositif de stockage (19) pour recevoir des filtres usagés (20).

4. Installation de filtre selon l'une quelconque des revendications 1 à 3, comprenant en outre un dispositif de changement (21), le dispositif de commande (11) étant en outre configuré pour transporter un filtre usagé (20) au moyen du dispositif de changement (21) du réceptacle de filtre (16) au dispositif de stockage (19) et un filtre non usagé (18) du magasin (17) au réceptacle de filtre (16).

5. Installation de filtre selon l'une quelconque des revendications précédentes, dans laquelle un second dispositif de mesure (9) est relié en aval du dispositif de filtre (4) et le second dispositif de mesure (9) est configuré pour déterminer une quantité de gaz anesthésique dans le mélange de gaz circulant hors du dispositif de filtre (4) .

6. Installation de filtre selon l'une quelconque des revendications précédentes, dans laquelle le dispositif de filtre (4) comprend un préfiltre (7) pour filtrer des particules et/ou l'humidité à partir du mélange de gaz.
